(19) Europäisches Patentamt
European Patent Office
Office européen des brevets

(11) Veröffentlichungsnummer: **0 166 183**
**B1**

(12) # EUROPÄISCHE PATENTSCHRIFT

(45) Veröffentlichungstag der Patentschrift: **03.10.90**

(51) Int. Cl.$^5$: **A 61 K 31/44**

(21) Anmeldenummer: **85106121.8**

(22) Anmeldetag: **18.05.85**

(54) **Verwendung von Hydroxyindolderivaten zur Herstellung eines Arzneimittels zur Senkung des Blutdrucks.**

(30) Priorität: **28.05.84 DE 3419935**

(43) Veröffentlichungstag der Anmeldung:
**02.01.86 Patentblatt 86/01**

(45) Bekanntmachung des Hinweises auf die Patenterteilung:
**03.10.90 Patentblatt 90/40**

(84) Benannte Vertragsstaaten:
**AT BE CH DE FR GB IT LI NL SE**

(56) Entgegenhaltungen:
**EP-A-0 007 399**

(73) Patentinhaber: **MERCK PATENT GESELLSCHAFT MIT BESCHRÄNKTER HAFTUNG**
**Frankfurter Strasse 250 Postfach 4119**
**D-6100 Darmstadt (DE)**

(72) Erfinder: **Hausberg, Hans-Heinrich, Dr.**
**Odenwaldstrasse 30**
**D-6105 Ober-Ramstadt (DE)**
Erfinder: **Böttcher, Henning, Dr.**
**Soderstrasse 95**
**D-6100 Darmstadt (DE)**
Erfinder: **Seyfried, Christoph, Dr.**
**Mathildenstrasse 6**
**D-6104 Seeheim-Jugenheim (DE)**
Erfinder: **Bergmann, Rolf, Dr.**
**Birkenhag 36**
**D-6101 Reichelsheim (DE)**

**EP 0 166 183 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Gegenstand der Erfindung ist die Verwendung von Hydroxyindolderivaten der Formel I

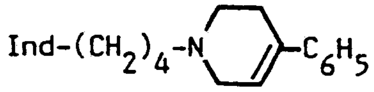

I

worin

Ind einen 4-, 5-, 6- oder 7-Hydroxyindol-3-ylrest bedeutet, der zusätzlich in 2-Stellung durch Alkyl mit 1—3 C-Atomen und/oder im Benzolring ein- oder zweifach durch Alkyl mit 1—3 C-Atomen, F, Cl, Br und/oder CN substituiert sein kann

sowie ihrer physiologisch unbedenklichen Säureadditionssalze s.o. Senkung des Blutdrucks.

Der Erfindung lag die Aufgabe zugrunde, Substanzen aufzufinden, die zur Senkung des Blutdrucks verwendet werden können. Diese Aufgabe wurde dadurch gelöst, daß man die oben genannten Verbindungen der Formel I sowie ihre physiologisch unbedenklichen Säureadditionssalze zur Herstellung eines Arzneimittels zur Senkung des Blutdrucks verwendet.

Die Verbindungen der Formel I und ihre Herstellung sind in der EP—A—0 007 399 beschrieben. Es finden sich dort jedoch keine Angaben über die hervorragende blutdrucksenkende Wirkung dieser Stoffe. So zeigen sie insbesondere blutdrucksenkende Wirkungen bei verschiedenen Formen des erhöhten Blutdrucks. Im einzelnen wird der bei katheter-tragenden (Methode vgl. J. R. Weeks und J. A. Jones, Proc. Soc. Exptl. Biol. Med. *104*, 646—648, 1960) wachen spontan hypertonen Ratten (Stamm SHR/NIH-MO (CHB-EMD) direkt gemessene arterielle Blutdruck nach einmaliger intragastraler Gabe in einem Dosisbereich von 0.3—100 mg/kg dosisabhängig gesenkt.

Weiterhin vermindern die Substanzen den an der Carotisschlinge des wachen mischrassigen Hundes gemessenen Blutdruck (Methodik vgl. F. H. Page, Science *89*, 273—274, 1939) im 10-Tage-Dauerversuch bei oraler Gabe von Dosen, die niedriger als 10 mg/kg liegen können, dosisabhängig auf ein erniedrigtes Niveau.

Auch an der narkotisierten Katze wurden die blutdrucksenkenden Wirkungen nach intravenöser Applikation von 0.1 mg/kg durch direkte Messung des Carotisdruckes verifiziert.

Der Rest Ind besitzt bevorzugt außer der 4-, 5-, 6- oder 7-Hydroxygruppe entweder keine weiteren Substituenten oder eine zusätzliche Methylgruppe oder ein zusätzliches Chloratom; er steht vorzugsweise für 5- oder 6-Hydroxyindol-3-yl, 2-Methyl-5- oder -6-hydroxyindol-3-yl, 5-Hydroxy-6-methylindol-3-yl, 5-Methyl-6-hydroxyindol-3-yl, 5-Chlor-6-hydroxyindol-3-yl, 5-Hydroxy-6-chlorindol-3-yl oder 4-Hydroxy-7-chlorindol-3-yl.

Von den Verbindungen der Formel I sind als blutdrucksenkend besonders bevorzugt:

3-[4-(4-Phenyl-1,2,3,6-tetrahydropyridyl)-butyl]-5-hydroxyindol (Hydrochlorid, F. 258°);

3-[4-(4-Phenyl-1,2,3,6-tetrahydropyridyl)-butyl]-6-hydroxyindol (Hydrochlorid, F. 271°);

2-Methyl-3-[4-(4-phenyl-1,2,3,6-tetrahydropyridyl)-butyl]-5-hydroxyindol (Hydrochlorid, F. 255—257°).

Die Verbindungen der Formel I können in an sich bekannter Weise hergestellt werden, z.B. nach den in der EP—A—0 007 399 angegebenen Verfahren oder analog dazu. Ein besonders bevorzugtes Verfahren ist die Spaltung entsprechender Methoxyindolderivate mit Hilfe von Diisobutylaluminiumhydrid in Toluol.

Die Verbindungen der Formel I und ihre physiologisch unbedenklichen Salze können zu pharmazeutischen Zubereitungen verarbeitet werden. Hierbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Diese Zubereintungen können als Arzneimittel in der Human- oder Veterinärmedizin eingesetzt werden. Als Trägersubstanzen kommen organische oder anorganische Stoffe in Frage, die sich für die enterale (z.B. orale) oder parenterale Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate. Die neuen Verbindungen können auch lyophilisiert und die erhaltenen Lyophilisate z.B. zur Herstellung von Injektionspräparaten verwendet werden. Die Zubereitungen können sterilisiert sein und/oder Hilfsstoffe enthalten, z.B. Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der therapeutischen Behandlung des menschlischen oder tierischen Körpers und bei der Bekämpfung von Krankheiten verwendet werden, insbesondere von Krankheiten, die mit zu hohem Blutdruck verbunden sind, z.B. vegetative Regulationsstörungen bei hypertoner Ausgangslage, periphere Durchblutungsstörungen, zerebrovaskuläre Insuffizienz, vaskuläre Kopfschmerzen einschließlich Migräne.

Dabei werden die erfindungsgemäßen Substanzen in der Regel in Analogie zu bekannten, im Handel befindlichen Präparaten (z.B. Dihydroergocristin) verabreicht, vorzugsweise in Dosierungen zwischen etwa 0,2 und 50 mg, insbesondere zwischen 1 und 10 mg pro Dosierungseinheit. Die tägliche Dosierung liegt vorzugsweise zwischen etwa 0,003 und 1 mg/kg Körpergewicht. Die niedrigen Dosierungen (etwa 0,2 bis 1 mg pro Dosierungseinheit; etwa 0,003 bis 0,01 mg/kg Körpergewicht) kommen dabei insbesondere für die Verwendung als Migränemittel in Betracht; für die übrigen Indikationen werden Dosierungen zwischen 1 und 10 mg pro Dosierungseinheit bevorzugt. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Herstellungsbeispiel

Zu einer Suspension von 3,46 g 2-Methyl-3-[4-(4-phenyl-1,2,3,6-tetrahydropyridyl)-butyl]-5-methoxy-indol in 30 ml Toluol tropft man unter Stickstoff 31 ml einer 20%igen Lösung von Diisobutylaluminiumhydrid in Toluol, kocht drei Stunden und kühlt ab. Nach üblicher Aufarbeitung erhält man 2-Methyl-3-[4-(4-phenyl-1,2,3,6-tetrahydropyridyl)-butyl]-5-hydroxyindol. Hydrochlorid, F. 255—257°.

Analog erhält man durch Spaltung der entsprechenden 4-Methoxyverbindung das 3-[4-(4-Phenyl-1,2,3,6-tetrahydropyridyl)-butyl]-4-hydroxy-7-chlorindol, F. 208—210°.

Die nachstehenden Beispiele betreffen pharmazeutische Zubereitungen, die Verbindungen der Formel I oder ihre Säureadditionssalze enthalten:

Beispiel A: Tabletten

Ein Gemisch von 1 kg 3-[4-(4-Phenyl-1,2,3,6-tetrahydropyridyl)-butyl]-5-hydroxy-indol-hydrochlorid, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

2 kg 2-Methyl-3-[4-(4-phenyl-1,2,3,6-tetrahydropyridyl)-butyl]-5-hydroxy-indol werden in üblicher Weise in Hartgelatinekapseln gefüllt, so daß jede Kapsel 10 mg des Wirkstoffs enthält.

Beispiel D: Ampullen

Ein Lösung von 1 kg 3-[4-(4-Phenyl-1,2,3,6-tetrahydropyridyl)-butyl]-6-hydroxyindol-hydrochlorid in 60 l zweifach destilliertem Wasser wird steril filtriert, in Ampullen abgefüllt, unter sterilen Bedingungen lyophilisiert und steril verschlossen. Jede Ampulle enthält 5 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

**Patentanspruch**

Verwendung von Hydroxyindolderivaten der Formel I

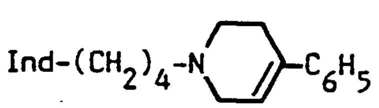

I

worin

Ind einen 4-, 5-, 6- oder 7-Hydroxyindol-3-ylrest bedeutet, der zusätzlich in 2-Stellung durch Alkyl mit 1—3 C-Atomen und/oder im Benzolring ein- oder zweifach durch Alkyl mit 1—3 C-Atomen, F, Cl, Br und/oder CN substituiert sein kann

und ihrer physiologisch unbedenklichen Säureadditionssalze zur Herstellung eines Arzneimittels zur Senkung des Blutdrucks.

**Revendication**

Utilisation de dérivés d'hydroxyindole de la formule I

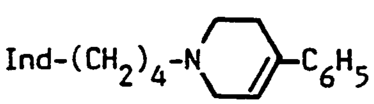

I

dans laquelle

Ind désigne un radical 4-, 5-, 6- ou 7-hydroxyindole-3-yle qui peut être en outre substitué dans la position 2 par un radical alkyle avec 1 à 3 atomes de C et/ou, dans l'anneau benzénique, une ou deux fois par un radical alkyle avec 1 à 3 atomes de C ou par du F, Cl, Br et/ou CN de même que leurs sels d'addition d'acide inoffensifs sur le plan physiologique pour la fabrication d'un médicament destiné à l'abaissement de la tension artérielle.

**Claim**

Use of hydroxyindole derivatives of the formula I

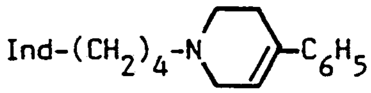

I

wherein

Ind denotes a 4-, 5-, 6- or 7-hydroxyindol-3-yl radical, which can additionally be substituted in the 2-position by alkyl with 1—3 C atoms and/or monosubstituted or disubstituted in the benzene ring by alkyl with 1—3 C atoms, F, Cl, Br and/or CN, and their physiologically acceptable acid addition salts for the preparation of a medicament for lowering blood pressure.